# EUROPEAN PATENT APPLICATION

(11) **EP 3 459 588 A1**
(43) Date of publication of application: **27.03.2019**
(21) Application number: 17306221.7
(22) Date of filing: 20.09.2017
(51) Int. Cl.: A61N 1/32, A61K 9/00, A61K 8/73

(54) **ELECTRICAL METHOD OF DELIVERING HYALURONIC ACID THROUGH THE SKIN**

(71) Applicant: L'OREAL, 75008 Paris (FR)
(72) Inventor: CAZARES DELGADILLO, Jennyfer, 94152 Chevilly La Rue (FR); KALIA, Yogeshwar N., 1211 Geneva 4 (CH); DEL RIO SANCHO, Sergio, 1211 Geneva 4 (CH); SERNA JIMENEZ, Cesar, 1211 Geneva 4 (CH); LAPTEVA, Maria, 1211 Geneva 4 (CH)
(74) Representative: Nony

(57) **Abstract**

The present invention relates to an electrical method of delivering hyaluronic acid through the skin, the electrical method comprising: applying a selected electroporation pulsatile current stimulus, from any device and/or support comprising at least one electrode, the electroporation pulsatile current stimulus being of a character and for a duration sufficient to transdermally deliver hyaluronic acid, for example an aqueous composition, to a biological subject, and transporting different rates of hyaluronic acid across the skin in accordance to the selected current mode.

## Description

The present invention relates to the field of skincare and/or the care of skin appendages. The term "skin and/or its appendages" is intended to mean in particular the skin, the mucous membranes, the lips, the scalp, the eyelashes, the eyebrows and the hair.

A subject of the invention is a cosmetic treatment method for the skin and/or appendages thereof, comprising at least one step consisting in applying at least one composition comprising at least one hyaluronic acid or a derivative thereof to the skin. The method of the invention is other than therapeutic.

The present invention also relates to a topical cosmetic, in particular a care cosmetic composition comprising, in a physiologically acceptable medium, at least one hyaluronic acid or a derivative thereof.

Hyaluronic acid and its various forms (e.g., hyaluronic acid salts, sodium hyaluronate, oxidized forms hyaluronic acid, anions of hyaluronic acid, and the like) may have one or more beneficial uses. For example, hyaluronic acid may act as an antiaging in humans. Hyaluronic acid is most abundant in the skin, accounting for 50% of the total hyaluronic acid of the human body and has the unique capacity to attach and retain large amounts of moisture. This highly contributes to the healthy and young appearance of the skin.

One problem, however, is that conventional hyaluronic acids use in topically applied anti-aging preparations, have molecules with high molecular weight (usually superior to 1,000 kDa) and diameter. Substances with molecular weight superior to 500 kDa cannot be absorbed into the skin. Conventional hyaluronic acids may be around 3µm in diameter, whereas the intercellular space is only 0.015 to 0.050 µm and just 0.006 to 0.010 µm at the hyaline membrane. This makes it impossible for conventional hyaluronic acids to penetrate passively into deep layers of the skin (Jegasothy et al., 2014; Desai et al., 2010).

Electroporation is a method used to transiently create aqueous pores in cell membranes, using electric pulses of high voltage and short duration. It has been used to enhance transdermal delivery of molecules with different lipophilicities and sizes including high molecular weight actives (proteins, peptides, and oligonucleotides).

As used herein, the term "electroporation" means the temporary creation of holes or pores by an applied electrical potential and through which high molecular cosmetic agents may pass. Electroporation is now widely used in biology, particularly for transfection studies, where plasmids, DNA fragments and other genetic material are introduced into living cells. During electroporation pulsing, molecules which are not normally skin permeant are able to pass through the skin during the period of induced reversible membrane permeabilization. The permeabilized state is caused by the generation of an electrical field in the cell suspension or tissue of sufficient field strength to perturb the cell surface membrane's proteolipid structure. During this short period of permeabilization, external agents can rapidly transfer across the surface membrane via these pores. With appropriate electrical parameters for the poration (field strength, pulse width, number of pulses etc), resealing of the membrane begins almost immediately after the pulsing. The surface membrane can reorganize with a full restoration of its former structural integrity, receptor status and other functional properties. Electrical fields for poration are commonly generated by capacitor discharge power units using pulses of very short (micro to millisecond) time course. Square wave and radio frequency pulses have also been used for cell electroporation.

Electroporation (also called electropermeabilization) may transitory change the structural perturbation of the lipid bilayer of the skin due to the application of relatively high voltage pulses, allowing a reduction in skin impedance and significant increase in skin deposition. It is a highly efficient strategy for the introduction of foreign high molecular weight substances into many cell types.

The main advantage of electroporation is its applicability for transient and stable transfection of all cell types. Furthermore, because electroporation is easy and rapid, it is able to transfect a large number of cells in a short time once optimum electroporation conditions are determined.

An electrical pulse at an optimized voltage and only lasting a few microseconds to a millisecond is discharged through the cell suspension. This disturbs the phospholipid bilayer of the membrane and results in the formation of temporary pores. The electric potential across the cell membrane simultaneously rises to allow charged molecules like DNA to be driven across the membrane through the pores in a manner similar to electrophoresis (Shigekawa and Dower, 1988).

It is known from WO 03/013615 an electroporative method for delivering a polynucleotide into cells of an organ.

It is also known from WO 2005/035755 a method of introducing nucleic acid into cells by electroporation, comprising the step of causing an electrode surface to support a nucleic acid and to effect adhesion of cells on the obtained nucleic acid supporting electrode surface.

WO 98/52613 discloses a method of administration of hyaluronic acid with an electrical assisted delivery method for treating a disease. The electrical current which is used is not specified.

There is a need for improving the delivering of moderate-high size compounds, such as hyaluronic acid, through the skin, without altering the skin.

### Electrical method

In some embodiments, an electrical method of delivering hyaluronic acid, for example an aqueous hyaluronic acid composition, through the skin, comprises applying a selected electroporation pulsatile current stimulus from any device and/or support, comprising at least one electrode, the electroporation pulsatile current stimulus being of a character and for a duration sufficient to transdermally deliver hyaluronic acid, for example an aqueous composition, to a biological subject, and transporting different rates of hyaluronic acid across the skin in accordance to the selected current mode.

The method according to the invention delivers hyaluronic acid through electroporation. This idea is at the basis of the present invention.

Nevertheless, in some embodiments, iontophoresis can be added to electroporation.

This method may allow significant changes in skin impedance and thus an increase permeability of the hyaluronic acid through keratinous material, and in particular through the skin.

The method of the invention is cosmetic and non-therapeutic.

In the invention, the selected current stimulus is applied to the keratinous material of the biological subject, in particular the skin.

In some embodiments, the administration of hyaluronic acid is conducted on live, human beings. In some embodiments, the administration of hyaluronic acid is conducted on any biological subject. In some embodiments, biological subjects include, but are not limited to, mammals, including human beings.

In some embodiments of the electrical method, applying an electroporation pulsatile current stimulus to a biological subject may include generating an electroporation pulsatile current stimulus having a voltage below 500 V, preferably below 400 V and more preferably below 300 V.

The given value of the voltage corresponds to the peak value of the voltage.

In some embodiments of the electrical method, applying an electroporation pulsatile current stimulus to a biological subject may include generating an electroporation pulsatile current stimulus having a voltage higher than 20 V, preferably higher than 50 V, and more preferably higher than 80 V and even higher than 100 V.

In an embodiment, the voltage may be of 250 V. In another embodiment, the voltage may be of 99.9 V.

Such electroporation pulsatile current stimulus may enable to reduce the impedance of keratinous material, and in particular of the skin. The reduction of the impedance of the skin may enable to allow the passage of moderate-high size compounds, such as hyaluronic acid.

In some embodiments, the electroporation pulsatile current stimulus may comprise pulses having a pulse duration ranging from 3 milliseconds to 100 milliseconds, preferably from 5 milliseconds to 70 milliseconds and more preferably from 7 milliseconds to 50 milliseconds.

In an embodiment, the pulse duration may be of 15 ms. In another embodiment, the pulse duration may be of 40 ms.

In some embodiments, the electroporation pulsatile current stimulus may comprise pulses having a pulse interval ranging from 3 milliseconds to 100 milliseconds, preferably from 5 milliseconds to 80 milliseconds and more preferably from 7 milliseconds to 60 milliseconds.

In an embodiment, the pulse interval may be of 50 ms.

In some embodiments, the electroporation pulsatile current stimulus may comprise a number of pulses ranging from 1 to 200 pulses, preferably from 3 to 150 pulses, and more preferably from 5 to 100 pulses.

In an embodiment, the number of pulses may be of 60 pulses. In another embodiment, the number of pulses may be of 6 or 9 pulses.

In an embodiment, the electroporation pulsatile current stimulus may comprise generating a non-constant current, which may comprise a very short pulsatile current.

In some embodiments, the electroporation pulsatile current stimulus may comprise generating a pulsed current having periodic square waveforms, rectangular waveforms, saw tooth waveforms, spiked waveforms, trapezoidal waveforms, triangle waveforms, or combinations thereof, and/or sinusoidal waveforms, non-sinusoidal waveforms, or combinations thereof. In an embodiment, the electroporation pulsatile current stimulus may have square waveforms or rectangular waveforms.

### OPTIONAL IONTOPHORESIS CURRENT

In some embodiments of the electrical method, the electrical method also comprises applying to a biological subject an iontophoresis direct current stimulus having an average current density ranging from 0.001 mA/cm² to 1 mA/cm², preferably from 0.01 mA/cm² to 0.5 mA/cm², and more preferably from 0.05 mA/cm² to 0.3 mA/cm², and in particular up to or below 0.2 mA/cm². The average current density may be above 0.2 mA/cm². The average current density may be up to 0.2 mA/cm².

The average current density is an average in time.

In some embodiments of the electrical method, applying a selected current stimulus to a biological subject includes generating a direct current stimulus having an average current density of 0.2 mA/cm².

In some embodiments of the electrical method, applying a selected current stimulus to a biological subject includes generating a direct current, which can be continuous or pulsatile direct current.

In some embodiments, the iontophoresis direct current stimulus is applied for a duration ranging from 1 minute to 60 minutes, preferably from 5 minutes to 50 minutes and more preferably from 7 minute to 40 minutes. In an embodiment, the duration of the iontophoresis direct current stimulus may be of 20 minutes. In an embodiment, the duration of the iontophoresis direct current stimulus may be of 10 minutes.

In some embodiments of the electrical method, applying a selected current stimulus to a biological subject includes generating a pulsed current having sinusoidal waveforms, non-sinusoidal waveforms, or combinations thereof.

In some embodiments of the electrical method, applying a selected current stimulus to a biological subject includes generating a pulsed current having periodic square waveforms, rectangular waveforms, saw tooth waveforms, spiked waveforms, trapezoidal waveforms, triangle waveforms, or combinations thereof.

In some embodiments of the electrical method, applying a selected current stimulus to a biological subject may include generating an electroporation pulsatile current stimulus as described above and an iontophoresis direct current stimulus as described above.

The combination of the electroporation pulsatile current stimulus and of the iontophoresis direct current stimulus such as described above enables to improve the decrease of the impedance of the skin, and enhance the passage of the hyaluronic acid through the skin.

In some embodiments of the electrical method, applying a selected current stimulus to a biological subject includes concurrently delivering direct current and the pulsatile current stimulus and generating a pulsed current stimulus having an average current density ranging from 0.005 mA/cm² to 0.5 mA/cm², in particular from 0.05 mA/cm² to 0.5 mA/cm²; a pulse duration ranging from 100 microseconds to 500 microseconds, in particular from 200 microseconds to 300 microseconds; and a pulse frequency ranging from 1 Hertz to 500 Hertz, in particular from 100 Hertz to 300 Hertz.

### HYALURONIC ACID

In the context of the present invention, the term "hyaluronic acid or a derivative thereof' covers in particular the basic unit of hyaluronic acid of formula:

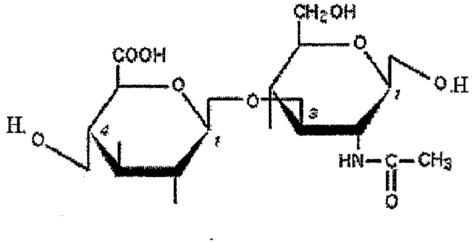

It is the smallest fraction of hyaluronic acid comprising a disaccharide dimer, namely D glucuronic acid and N acetylglucosamine.

The term "hyaluronic acid or a derivative thereof' also comprises, in the context of the present invention, the linear polymer comprising the polymeric unit described above, linked together in the chain via alternating β(1,4) and β(1,3) glycosidic linkages, having a molecular weight (MW) that can range between 380 and 13 000 000 daltons. This molecular weight depends in large part on the source from which the hyaluronic acid is obtained and/or on the preparation methods.

The term "hyaluronic acid or a derivative thereof' also comprises, in the context of the present invention, the hyaluronic acid salts, and in particular the alkali metals salts such as the sodium salt and the potassium salt.

In the natural state, hyaluronic acid is present in pericellular gels, in the base substance of the connective tissues of vertebrate organs such as the dermis and epithelial tissues, and in particular in the epidermis, in the synovial fluid of the joints, in the vitreous humor, in the human umbilical cord and in the crista galli apophysis.

Hyaluronic acid is a predominant glucosaminoglycan found in the skin. Thus, the fibroblasts synthesize predominantly collagens, matrix glycoproteins other than collagens (fibronectin, laminin), proteoglycans and elastin. The keratinocytes, for their part, synthesize predominantly sulfated glycosaminoglycans and hyaluronic acid. Hyaluronic acid is also called hyaluronan (HA).

Hyaluronic acid is present in the free state in the epidermis and in the dermis and is responsible for turgescence of the skin. This polysaccharide can in fact retain a large volume of water, corresponding to up to 1000 times its weight. In this sense, hyaluronic acid plays an important role in increasing the amounts of water bound in the tissue, and also in the mechanical properties of the skin and in wrinkle formation.

Thus, the term "hyaluronic acid or a derivative thereof' comprises all the fractions or subunits of hyaluronic acid having a molecular weight in particular within the molecular weight range recalled above.

In the context of the present invention, hyaluronic acid fractions which do not have an inflammatory activity are preferably used.

By way of illustration of the various hyaluronic acid fractions, reference may be made to the document "Hyaluronan fragments: an information-rich system", R. Stern et al., European Journal of Cell Biology 58 (2006) 699 715, which reviews the listed biological activities of hyaluronic acid according to its molecular weight.

According to an embodiment of the invention, the hyaluronic acid has a molecular weight in a range from 1 Da to 4000 kDa, preferably from 1 kDa to 2000 kDa and more preferably from 5 kDa to 200 kDa.

According to a preferred embodiment of the invention, the hyaluronic acid fractions suitable for the use covered by the present invention have a molecular weight of between 800 Da and 2 000 000 Da, preferably between 20 000 and 1 000 000 Da, in particular between 50 000 and 500 000, especially between 100 000 and 200 000 Da.

The hyaluronic acid may have a molecular weight in a range from 800 Da to 2000 kDa, preferably from 20 kDa to 1000 kDa and more preferably from 50 kDa to 500 kDa, even better from 100 kDa to 200 kDa. In an embodiment, the molecular weight of the hyaluronic acid is of 120 000 Da. In this case, the term used is intermediate-molecular-weight hyaluronic acid.

Finally, the term "hyaluronic acid or a derivative thereof' also comprises hyaluronic acid esters in particular those in which all or some of the carboxylic groups of the acid functions are esterified with oxyethylenated alkyls or alcohols, containing from 1 to 20 carbon atoms, in particular with a degree of substitution at the level of the D glucuronic acid of the hyaluronic acid ranging from 0.5 to 50%.

Mention may in particular be made of methyl, ethyl, n propyl, n pentyl, benzyl and dodecyl esters of hyaluronic acid. Such esters have in particular been described in D. Campoccia et al. "Semisynthetic resorbable materials from hyaluronan esterification", Biomaterials 19 (1998) 2101 2127.

The molecular weights indicated above are also valid for the hyaluronic acid esters.

Hyaluronic acid may in particular be hyaluronic acid supplied by the company Hyactive under the trade name CPN (MW: 10 to 150 kDa), by the company Soliance under the trade name Cristalhyal (MW: 1.1 × 106), by the company Bioland under the name Nutra HA (MW: 820 000 Da), by the company Bioland under the name Nutra AF (MW: 69 000 Da, by the company Bioland under the name Oligo HA (MW: 6100 Da) or else by the company Vam Farmacos Metica under the name D Factor (MW: 380 Da).

In one embodiment, the hyaluronic acid is present in the form of spheres. In particular, such spheres are sold by the company BASF under the name Sphere d'Acide Hyaluronique [hyaluronic acid sphere]. It is a mixture of hyaluronic acid of various molecular weights, i.e. of MW 1.5 × 106, 400 000 and 600 000 Da.

In some embodiments of the electrical method, the method further comprises transdermally delivering a composition including one or more of hyaluronic acid, hyaluronic acid derivatives, ions of hyaluronic acid, and ions of hyaluronic acid derivatives.

The one or more of hyaluronic acid, hyaluronic acid derivatives, ions of hyaluronic acid, and ions of hyaluronic acid derivatives may be present in amounts ranging from 0.01 % to 100 % by weight, preferably from 0.5 % to 60 % by weight.

The amounts by weight are given relative to the total weight of the composition.

The hyaluronic acid or a derivative thereof may be present in the composition according to the present invention at a content of between 0.001% and 20%, preferably between 0.01% and 10%, and more particularly between 0.01% and 5% by weight, relative to the total weight of the composition.

In an embodiment, the composition comprises 1% by weight of hyaluronic acid.

In an embodiment, the composition comprises 1% by weight of hyaluronic acid, and water.

In an embodiment, the hyaluronic acid may be sodium hyaluronate.

In an embodiment, the molecular weight of the hyaluronic acid may be of 120 kDa.

In some embodiments of the electrical method, the method further comprises transdermally delivering a composition, for example an aqueous composition, including, one or more of hyaluronic acid, hyaluronic acid derivatives, ions of hyaluronic acid, and ions of hyaluronic acid derivatives present in amounts ranging from 0.1 % to 20 % by weight; one or more silicon surface-active agents present in amounts ranging from 0.01 % to 30 % by weight; one or more ionic polymers present in amounts ranging from 0.01 % to 10% by weight; and water present in an amount of at least 30 % by weight.

In some embodiments of the electrical method, the method further comprises
transdermally delivering an aqueous composition including,
one or more silicon surface-active agents present in amounts ranging from 0.01 % to 30 % by weight;
one or more ionic polymers present in amounts ranging from 0.01 % to 10 % by weight; and
water present in an amount of at least 30 % by weight.

In some embodiments of the electrical method, the method further comprises transdermally delivering an aqueous composition including, one or more of hyaluronic acid, hyaluronic acid derivatives, ions of hyaluronic acid, and ions of hyaluronic acid derivatives present in amounts ranging from 0.01 % to 30 % by weight; one or more silicon surface-active agents present in amounts ranging from 0.01 % to 30 % by weight; one or more non-ionic polymers present in amounts ranging from 0.01 % to 20 % by weight; and water present in an amount of at least 30 % by weight.

In some embodiments of the electrical method, transdermally delivering hyaluronic acid, in particular the aqueous active agent composition includes, generating a direct current stimulus having an average current density ranging from 0.01 mA/cm² to 0.5 mA/cm²; and generating a pulsed current stimulus having an average current density ranging from 0.01 mA/cm² to 10 mA/cm²; a pulse duration ranging from 10 microseconds to 500 microseconds; and a pulse frequency ranging from 10 Hertz to 500 Hertz; the direct current and the pulsed current of a duration sufficient to transdermally deliver an aqueous active agent composition to a biological subject.

In some embodiments of the electrical method, the method comprises a step of measuring at least one of the temperature of the skin, the impedance of the skin, and a pH of the composition.

In some embodiments of the electrical method, the application of current stimulus is reduced to a safety level when a measured value measured by one of the sensors exceeds a safety range or a safety value.

In some embodiments of the electrical method, the method comprises a step of measuring the pH of the composition. When the measured pH exceeds a pH safety range, the application of current stimulus is switched to a safety level, for example a safety level less than IV, such as 0,5V. The pH safety range may be pH 4 to 7. In some embodiment, when the measured pH exceeds a pH safety range, for example the range from 4 to 7, the device switches the polarity during a short time to enable to reequilibrate the pH.

In some embodiments of the electrical method, the method comprises a step of measuring the impedance of the skin. When the measured impedance exceeds an impedance safety range, the application of current stimulus is reduced to a safety level to avoid adverse event. The safety level may be less than IV, such as 0,5V. The impedance safety range may be 50 Ω to 1 MΩ.

In some embodiments of the electrical method, the method comprises a step of measuring the temperature of the skin. When the measured temperature exceeds a temperature safety value, the application of current stimulus is switched to a safety level, for example less than IV, such as 0,5V. The temperature safety value may be chosen less than 42°C.

In a preferred embodiment of the electrical method, the method comprises the steps of:
- measuring the temperature of the skin, and
- measuring the impedance with the composition, and
- measuring the pH of the composition.

Then, the device is configured for treating the results and regulating the microcurrent, and polarity in the case of combination with iontophoresis.

### Composition

The present invention also relates to a topical cosmetic, in particular a care cosmetic, composition comprising, in a physiologically acceptable medium, at least one hyaluronic acid or a derivative thereof.

In some embodiments, the invention also relates to an electrical composition comprising one or more of hyaluronic acid, hyaluronic acid derivatives, ions of hyaluronic acid, and ions of hyaluronic acid derivatives present in amounts ranging from 0.005 % to 30 % by weight, preferably in an amounts ranging from 0.01 % to 20 % by weight, 0.05 % to 10 % by weight.

The composition may be deprived of water.

In some embodiments, the invention also relates to an electrical composition comprising
one or more of hyaluronic acid, hyaluronic acid derivatives, ions of hyaluronic acid, and ions of hyaluronic acid derivatives present in amounts ranging from 0.01 % to 30 % by weight, preferably in an amounts ranging from 0.1 % to 20 % by weight, 0.1% to 5 % by weight, and
water present in an amount of at least 0 % by weight, preferably in an amount of at least 10 % by weight, more preferably in an amount of at least 60 % by weight, more preferably in an amount of at least 90 % by weight.

In some embodiments, the invention also relates to an electrical composition comprising
one or more of hyaluronic acid, hyaluronic acid derivatives, ions of hyaluronic acid, and ions of hyaluronic acid derivatives present in amounts ranging from 0.005 % to 30 % by weight, preferably in an amounts ranging from 0.01 % to 20 % by weight, 0.05 % to 10 % by weight, and
water present in an amount of at least 0 % by weight, preferably in an amount of at least 10 % by weight, more preferably in an amount of at least 60 % by weight.

In particular, the composition of the invention may be for improving the barrier function of the skin. It can thus be used in hydrating the skin, in improving the suppleness of the skin, in improving and/or decreasing the microrelief of the skin, and also in combating the signs of aging of the skin.

In some embodiments of the electrical composition, the composition comprises one or more of hyaluronic acid, hyaluronic acid derivatives, ions of hyaluronic acid, and ions of hyaluronic acid derivatives present in amounts ranging from 0.1 % to 30 % by weight; one or more silicon materials present in amounts ranging from 0.1 % to 30 % by weight; and water present in an amount of at least 20 % by weight; the electrical composition having an aqueous phase that is at least 30% by weight relative to the total weight of the electrical composition.

In some embodiments of the electrical composition, the composition further comprises one or more ionic polymers present in amounts ranging from 0.01 % to 10% by weight; wherein the one or more of hyaluronic acid, hyaluronic acid derivatives, ions of hyaluronic acid, and ions of hyaluronic acid derivatives are present in amounts ranging from 0.1 % to 30 % by weight.

In some embodiments of the electrical composition, the composition further comprises one or more non-ionic polymers present in amounts ranging from 0.01 % to 20% by weight; wherein the one or more of hyaluronic acid, hyaluronic acid derivatives, ions of hyaluronic acid, and ions of hyaluronic acid derivatives are present in amounts ranging from 0.01 % to 30 % by weight.

In some embodiments of the electrical composition, the composition further comprises a pH ranging from 2 to 7.5.

In some embodiment, when the measured pH exceeds a pH safety range, for example the range from 4 to 7, the device switches the polarity during a short time to enable to reequilibrate the pH.

### Electrical kit

In some embodiments, the invention also provides an electrical kit comprising:
- an electrical composition including one or more of hyaluronic acid, hyaluronic acid derivatives, ions of hyaluronic acid, and ions of hyaluronic acid derivatives, and
- an electrical device for carrying the electrical method as described above.

The composition may be an aqueous composition.

The electrical kit may be configured such that hyaluronic acid and water are already mixed in the composition when the composition is applied to the skin.

In some embodiments, the electrical device may comprise at least one of a temperature sensor, an impedance sensor, and a pH sensor. The electrical device may comprise at least two of a temperature sensor, an impedance sensor, and a pH sensor. In an embodiment, the electrical device may comprise a temperature sensor, an impedance sensor, and a pH sensor.

The device may be configured such that the application of current stimulus is reduced to a safety level when a measured value measured by one of the sensors exceeds a safety range or a safety value.

In a preferred embodiment, the method, the composition and the kit above enable to treat wrinkles and ageing signs, to improve smoothness, quality of skin and appearance of the skin. It can thus be used in hydrating the skin, in improving the suppleness of the skin, in improving and/or decreasing the microrelief of the skin, and also in combating the signs of aging of the skin.

In another embodiment, the method is used to minimize skin anti-aging, and/or pigmentation, and/or volume, and/ or sagging wrinkle, and/or event tone and/or spots, and/or to improve firmness, and/or radiance, and/or smoothness, and/or softness of the skin. The method of the invention may be associated with the application of active agents associated to milli current (mcurrent) and/or micro current (µcurrent).

### DESCRIPTION OF THE DRAWINGS

The foregoing aspects and many of the attendant advantages of the disclosed subject matter will become more readily appreciated as the same become better understood by reference to the following detailed description, when taken in conjunction with the accompanying drawings, wherein:
FIGURE 1 is a flow diagram of a method in accordance with one embodiment;
FIGURE 2 is a flow diagram of a method in accordance with one embodiment.
FIGURES 3a and 3b are graphs showing the impedance change and the relative impedance for the tested treatments;
FIGURE 4 is a graph showing the skin deposition of 120kDa FL-HA formulation;
FIGURE 5 is a graph showing the comparison of skin deposition of 120kDa FL-HA formulation after electroporation alone or electroporation plus iontophoresis; and FIGURE 6 is a view of the skin images obtained.

### DETAILED DESCRIPTION

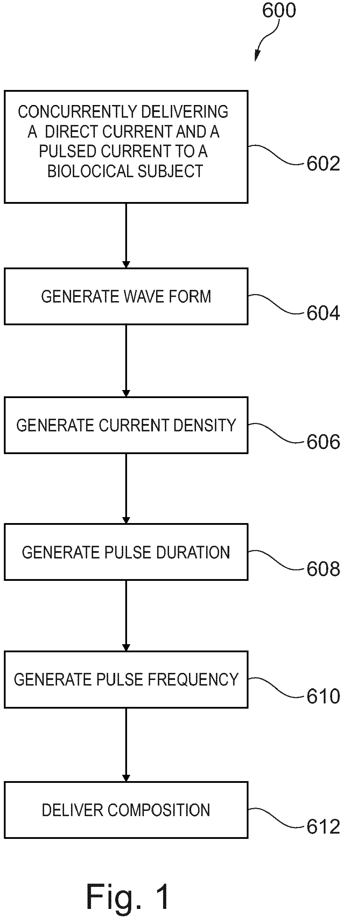

FIGURE 1 illustrates embodiments of a method 600 for delivering a cosmetic composition through the generation of electrical stimuli. In some embodiments, the method includes a step 602 for concurrently delivering a direct current and an electroporation pulsatile current to a biological subject, the direct current and the pulsed current is of a character and for a duration sufficient to deliver a cosmetic composition to the biological subject.

In some embodiments, the illustrated steps 604, 606, 608, and 610 are optional. Further, in some embodiments, the sequence of the steps 604, 606, 608, and 610 can be in any order and is not confined to the illustration. In some embodiments, the method 600 includes a step 604 for generating waveforms. In some embodiments, the user makes selections that cause the electrical device to generate the selected waveform or waveforms that constitute the electrical stimuli. In some embodiments, the method 600 includes a step 606 for generating current density. In some embodiments, the user makes selections that cause the electrical device to generate the selected current density. In some embodiments, the method 600 includes a step 608 for generating pulse duration. In some embodiments, the user makes selections that cause the electrical device to generate the selected pulse duration. In some embodiments, the method 600 includes a step 610 for generating pulse frequency. In some embodiments, the user makes selections that cause the electrical device to generate the selected pulse frequency.

In some embodiments, the method 600 for concurrently delivering the direct current and the pulsed current to a biological subject includes generating an electroporation pulsatile current stimulus having a voltage below 500 V, preferably below 250 V and more preferably below 200 V. The voltage may be higher than 20 V, preferably higher than 50 V, and more preferably higher than 100 V.

In some embodiments, the electroporation pulsatile current stimulus may comprise pulses having a pulse duration ranging from 5 milliseconds to 100 milliseconds, preferably from 7 milliseconds to 70 milliseconds and more preferably from 10 milliseconds to 50 milliseconds.

In some embodiments, the electroporation pulsatile current stimulus may comprise pulses having a pulse interval ranging from 5 milliseconds to 100 milliseconds, preferably from 7 milliseconds to 80 milliseconds and more preferably from 10 milliseconds to 60 milliseconds.

In some embodiments, the method 600 includes generating an the electroporation pulsatile current stimulus may comprise generating a pulsed current having periodic square waveforms, rectangular waveforms, saw tooth waveforms, spiked waveforms, trapezoidal waveforms, triangle waveforms, or combinations thereof, and/or sinusoidal waveforms, non-sinusoidal waveforms, or combinations thereof. In an embodiment, the electroporation pulsatile current stimulus may have square waveforms or rectangular waveforms.

In some embodiments, the method 600 for concurrently delivering the direct current and the pulsed current to a biological subject includes generating a direct current stimulus having an average current density ranging from 0.01 mA/cm² to 0.5 mA/cm², preferably from 0.01 mA/cm² to 0.4 mA/cm², and more preferably from 0.05 mA/cm² to 0.2 mA/cm².

In some embodiments, the method 600 for concurrently delivering the direct current and the pulsed current to a biological subject includes generating a direct current stimulus having an average current density of 0.2 mA/cm².

In some embodiments, the method 600 for concurrently delivering the direct current and the electroporation pulsatile current to a biological subject includes generating a pulsed current stimulus having an average current density ranging from 0.01 mA/cm² to 10 mA/cm², a pulse duration ranging from 50 microseconds to 1 milliseconds, and a pulse frequency ranging from 10 Hertz to 500 Hertz, and a duty cycle of pulses ranging from 1% to 90%.

In some embodiments, the method 600 for concurrently delivering the direct current and the pulsed current to a biological subject includes generating a pulsed alternating current stimulus having an average current density of 0.2 mA/cm², a pulse duration of 500 microseconds, and a pulse frequency of 200 Hertz.

In some embodiments, the method 600 for concurrently delivering the direct current and the pulsed current to a biological subject includes generating a pulsed current having an average current density ranging from 0.01 mA/cm² to 10 mA/cm², a pulse width ranging from 50 microseconds to 1 milliseconds, at least one wave packet (or wave train) ranging from 2 to 20 pulses, a frequency of wave packets ranging from 10 Hertz to 500 Hertz, and a duty of pulses ranging from 1% to 90%.

In some embodiments, the method 600 for concurrently delivering the direct current and the pulsed current to a biological subject includes generating a pulsed current stimulus having an average current density ranging from 0.01 mA/cm² to 10 mA/cm², a pulse width ranging from 50 microseconds to 1 milliseconds, at least one wave packet (wave train) having from 2 to 20 pulses with alternating polarity, a frequency of wave packets ranging from 10 Hertz to 500 Hertz, and a duty cycle of pulses ranging from 1% to 90%.

In some embodiments, the method 600 for concurrently delivering the direct current and the pulsed current to a biological subject includes generating a pulsed current having sinusoidal waveforms, non-sinusoidal waveforms, or combinations thereof.

In some embodiments, the method 600 for concurrently delivering the direct current and the pulsed current to a biological subject includes generating a pulsed current having periodic square waveforms, rectangular waveforms, saw tooth waveforms, spiked waveforms, trapezoidal waveforms, triangle waveforms, or combinations thereof.

In some embodiments, the method 600 comprises delivering a cosmetic composition chosen from a face care or body care composition, comprising in particular, an active agent chosen from humectant or moisturizing active agents, anti-ageing active agents, for example depigmenting active agents, active agents that act on cutaneous microcirculation, or seboregulating active agents, or a composition for making up the face or body.

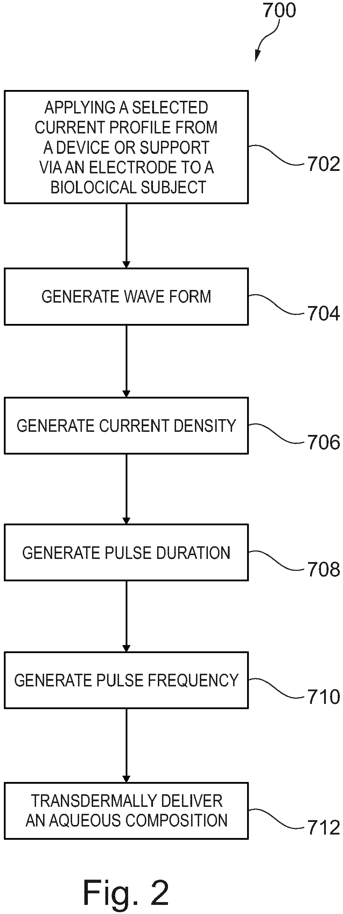

FIGURE2 illustrates embodiments of a method 700 for delivering an aqueous active agent composition, such as an aqueous hyaluronic acid composition through the skin to a biological subject through the generation of electrical stimuli of certain waveform types.

In some embodiments, the method 700 includes step 702 for applying a selected current stimulus, either direct current, pulsed current or a combination of both, from any device and/or support comprising at least one electrode to a biological subject, the direct current, the pulsed current or the combination of both is of a character and for a duration sufficient to transdermally deliver an aqueous composition to a biological subject, thus, transporting different rates of hyaluronic acid across the skin in accordance to the selected current mode.

In some embodiments, the illustrated steps 704, 706, 708, and 710 are optional. Further, in some embodiments, the sequence of the steps 704, 706, 708, and 710 can be in any order and is not confined to the illustration. In some embodiments, the method 700 includes a step 704 for generating waveforms. In some embodiments, the user makes selections that cause the electrical device to generate the selected waveform or waveforms that constitute the electrical stimuli. In some embodiments, the method 700 includes a step 706 for generating current density. In some embodiments, the user makes selections that cause the electrical device to generate the selected current density. In some embodiments, the method 700 includes a step 708 for generating pulse duration. In some embodiments, the user makes selections that cause the electrical device to generate the selected pulse duration. In some embodiments, the method 700 includes a step 710 for generating pulse frequency. In some embodiments, the user makes selections that cause the electrical device to generate the selected pulse frequency. In step 712, the method 700 includes transdermally delivering an aqueous composition.

In some embodiments, the method 700 for concurrently delivering the direct current and the pulsed current to a biological subject includes generating an electroporation pulsatile current stimulus having a voltage below 500 V, preferably below 250 V and more preferably below 200 V. The voltage may be higher than 20 V, preferably higher than 50 V, and more preferably higher than 100 V.

In some embodiments, the electroporation pulsatile current stimulus may comprise pulses having a pulse duration ranging from 5 milliseconds to 100 milliseconds, preferably from 7 milliseconds to 70 milliseconds and more preferably from 10 milliseconds to 50 milliseconds.

In some embodiments, the electroporation pulsatile current stimulus may comprise pulses having a pulse interval ranging from 5 milliseconds to 100 milliseconds, preferably from 7 milliseconds to 80 milliseconds and more preferably from 10 milliseconds to 60 milliseconds.

In some embodiments, the method 700 includes generating an electroporation pulsatile current stimulus may comprise generating a pulsed current having periodic square waveforms, rectangular waveforms, saw tooth waveforms, spiked waveforms, trapezoidal waveforms, triangle waveforms, or combinations thereof, and/or sinusoidal waveforms, non-sinusoidal waveforms, or combinations thereof. In an embodiment, the electroporation pulsatile current stimulus may have square waveforms or rectangular waveforms.

In some embodiments of the method 700 of delivering an aqueous hyaluronic acid composition through the skin, applying a selected current stimulus to a biological subject includes generating a direct current stimulus having an average current density ranging from 0.01 mA/cm² to 0.5 mA/cm², preferably from 0.01 mA/cm² to 0.4 mA/cm², and more preferably from 0.05 mA/cm² to 0.3 mA/cm².

In some embodiments of the method 700 of delivering an aqueous hyaluronic acid composition through the skin, applying a selected current stimulus to a biological subject includes generating a direct current stimulus having an average current density of 0.2 mA/cm².

In some embodiments of the method 700 of delivering an aqueous hyaluronic acid composition through the skin, applying a selected current stimulus to a biological subject includes generating a pulsed current having sinusoidal waveforms, non-sinusoidal waveforms, or combinations thereof.

In some embodiments of the method 700 of delivering an aqueous hyaluronic acid composition through the skin, applying a selected current stimulus to a biological subject includes generating a pulsed current having periodic square waveforms, rectangular waveforms, saw tooth waveforms, spiked waveforms, trapezoidal waveforms, triangle waveforms, or combinations thereof.

In some embodiments of the method 700 of delivering an aqueous hyaluronic acid composition through the skin, applying a selected current stimulus to a biological subject includes concurrently delivering the direct current and the pulsed current and generating a pulsed current stimulus having an average current density ranging from 0.05 mA/cm² to 0.5 mA/cm²; a pulse duration ranging from 200 microseconds to 300 microseconds; and a pulse frequency ranging from 100 Hertz to 300 Hertz.

In some embodiments, the method 700 of delivering an aqueous hyaluronic acid composition through the skin further comprises transdermally delivering a composition including one or more of hyaluronic acid, hyaluronic acid derivatives, ions of hyaluronic acid, and ions of hyaluronic acid derivatives present in amounts ranging from 0.01 % to 100 % by weight, preferably from 0.5 % to 60 % by weight.

In some embodiments, the method 700 of delivering an aqueous hyaluronic acid composition through the skin, further comprises transdermally delivering an aqueous composition including hyaluronic acid having a molecular weight in a range from 0.8 kDa to 2000 kDa, preferably from 20 kDa to 1000 kDa and more preferably from 50 kDa to 500 kDa, and in particular from 100 kDa to 200 kDa. In an embodiment, the molecular weight of the hyaluronic acid may be of 120 kDa.

In some embodiments, the method 700 of delivering an aqueous hyaluronic acid composition through the skin further comprises transdermally delivering an aqueous composition including, one or more of hyaluronic acid, hyaluronic acid derivatives, ions of hyaluronic acid, and ions of hyaluronic acid derivatives present in amounts ranging from 0.1 % to 20 % by weight; one or more silicon surface-active agents present in amounts ranging from 0.01 % to 30 % by weight; one or more ionic polymers present in amounts ranging from 0.01 % to 10% by weight; and water present in an amount of at least 30 % by weight.

In some embodiments, the method 700 of delivering an aqueous hyaluronic acid composition through the skin, further comprises transdermally delivering an aqueous composition including, one or more of hyaluronic acid, hyaluronic acid derivatives, ions of hyaluronic acid, and ions of hyaluronic acid derivatives present in amounts ranging from 0.01 % to 30 % by weight; one or more silicon surface-active agents present in amounts ranging from 0.01 % to 30 % by weight; one or more non-ionic polymers present in amounts ranging from 0.01 % to 20 % by weight; and water present in an amount of at least 30 % by weight.

In some embodiments of the method 700 of delivering an aqueous hyaluronic acid composition through the skin, transdermally delivering the aqueous active agent composition includes generating a direct current stimulus having an average current density ranging from 0.01 mA/cm² to 0.5 mA/cm²; and generating a pulsed current stimulus having an average current density ranging from 0.01 mA/cm² to 10 mA/cm²; a pulse duration ranging from 10 microseconds to 500 microseconds; and a pulse frequency ranging from 10 Hertz to 500 Hertz; the direct current and the pulsed current of a duration sufficient to transdermally deliver an aqueous active agent composition to a biological subject.

An electrical composition for use with the electrical methods described above in relation to FIGURES 1 and 2 is disclosed.

In some embodiments, the electrical composition comprises one or more of hyaluronic acid, hyaluronic acid derivatives, ions of hyaluronic acid, and ions of hyaluronic acid derivatives present in amounts ranging from 0.1 % to 30 % by weight, preferably in an amounts ranging from 0.5 % to 20 % by weight, 0.7 % to 10 % by weight, and water present in an amount of at least 20 % by weight, preferably in an amount of at least 40 % by weight, more preferably in an amount of at least 60 % by weight.

In some embodiments, the electrical composition includes one or more of hyaluronic acid, hyaluronic acid derivatives, ions of hyaluronic acid, and ions of hyaluronic acid derivatives present in amounts ranging from 0.1 % to 30 % by weight; one or more silicon materials present in amounts ranging from 0.1 % to 30 % by weight; and water present in an amount of at least 20 % by weight; the electrical composition having an aqueous phase that is at least 30% by weight relative to the total weight of the electrical composition.

In some embodiments, the electrical composition further comprises one or more ionic polymers present in amounts ranging from 0.01 % to 10% by weight; wherein the one or more of hyaluronic acid, hyaluronic acid derivatives, ions of hyaluronic acid, and ions of hyaluronic acid derivatives are present in amounts ranging from 0.1 % to 30 % by weight.

In some embodiments, the electrical composition further comprises one or more non-ionic polymers present in amounts ranging from 0.01 % to 20% by weight; wherein the one or more of hyaluronic acid, hyaluronic acid derivatives, ions of hyaluronic acid, and ions of hyaluronic acid derivatives are present in amounts ranging from 0.01 % to 30 % by weight.

In some embodiments, the electrical composition further comprises a pH ranging from 2 to 7.5.

In some embodiments of the electrical composition, the one or more silicon materials include one or more silicon surface-active agents. In some embodiments of an electrical composition, the silicon-containing surface active agents are selected from polydimethylsiloxane, poly[oxy(dimethylsilylane)], polyvinyl siloxane, cyclohexasiloxane, derivatives thereof, or any combination thereof.

In some embodiments of the electrical composition, the ionic polymers and nonionic polymers are selected from acrylonitrile/methyl methacrylate/vinylidene chloride copolymer, biosaccharide gum-1, sodium styrene/maleic anhydride copolymer, xanthan gum, ammonium polyacryloyldimethyl taurate, derivatives thereof, their ions, and any combination thereof.

In some embodiments of the electrical composition, the composition further comprises a vitamin, a fat, a solvent, a humectant, a viscosity reducer, a preservative, a chelating agent, a viscosity controller, a skin conditioner, an emollient, an emulsifier, a cleansing agent, an emulsion stabilizer, a viscosity increaser, an antioxidant, a binder, a skin bleaching agent, a pH adjuster, a buffering agent, a denaturant, a bulking agent, an opacifying agent.

In some embodiments of the electrical composition, the composition includes ionic polymers and nonionic polymers selected from biosaccharide gum-1 (and) sodium levulinate (and) glyceryl caprylate (and) sodium anisate, acrylates/c10-30 alkyl acrylate crosspolymer, carbomer, sodium styrene/maleic anhydride copolymer, nylon-12, xanthan gum, derivatives thereof, their ions, or any combination thereof.

In some embodiments, the electrical composition has a pH from 2 to 7.4.

In some embodiments, the electrical composition has a pH from 2 to 7.

In some embodiments, the electrical composition has a pH from 5.7 to 6.3.

In some embodiments, the electrical composition has a pH from 2 to 6.3.

In some embodiments, the electrical composition includes one or more vitamins selected from vitamin B5, vitamin A, vitamin B3, and vitamin E.

In some embodiments, the electrical composition includes one or more fats selected from nut oils, seed oils, and plant oils.

In some embodiments, the electrical composition includes one or more solvents selected from water, deionized water, and Eau de la Roche-Posay™.

In some embodiments, the electrical composition includes one or more humectants selected from glycerin, caprylyl glycol, and sodium hyaluronate.

In some embodiments, the electrical composition includes one or more viscosity reducers selected from glycerine.

In some embodiments, the electrical composition includes one or more preservatives selected from phenoxyethanol, salicylic acid, and sodium methylparaben.

In some embodiments, the electrical composition includes one or more chelating agents selected from disodium EDTA.

In some embodiments, the electrical composition includes one or more viscosity controllers selected from disodium EDTA, ammonium polyacryldimethyltauramide, and nylon-12.

In some embodiments, the electrical composition includes one or more skin conditioners selected from C12-15 alkyl benzoate, caprylyl glycol, glyceryl stearate and polyethylene glycol 100 Stearate, tocopheryl acetate, sodium hyaluronate, ethylhexyl palmitate, dimethicone and dimethiconol, dimethicone, dimethicone and dimethicone/vinyl dimethicone crosspolymer, biosaccharide gum-1, oxothiazolidinecarboxylic acid, ascorbic acid, sodium styrene/maleic anhydride copolymer, salicylic acid, cyclohexasiloxane, hydrogenated polyisobutene, biosaccharide gum-1 and sodium levulinate and glyceryl caprylate and sodium anisate, lemon extract, alcohol and Gentiana lutea root extract, and dimethicone and polyethylene glycol/polypropylene glycol-18/18 dimethicone.

In some embodiments, the electrical composition includes one or more emollients selected from C12-15 alkyl benzoate, caprylyl glycol, glyceryl stearate and polyethylene glycol 100 Stearate, ethylhexyl palmitate, dimethicone and dimethiconol, dimethicone, dimethicone and dimethicone/vinyl dimethicone crosspolymer, cyclohexasiloxane, hydrogenated polyisobutene, biosaccharide gum-1 and sodium levulinate and glyceryl caprylate and sodium anisate, and dimethicone and polyethylene glycol/polypropylene glycol-18/18 dimethicone.

In some embodiments, the electrical composition includes one or more emulsifiers selected from glyceryl stearate and polyethylene glycol 100 Stearate, cetyl alcohol, xanthan gum, triethanolamine, biosaccharide gum-1 and sodium levulinate and glyceryl caprylate and sodium anisate, and dimethicone and polyethylene glycol/polypropylene glycol-18/18 dimethicone.

In some embodiments, the electrical composition includes one or more cleansing agents selected from glyceryl stearate and polyethylene glycol 100 Stearate.

In some embodiments, the electrical composition includes one or more stabilizers selected from cetyl alcohol, xanthan gum, ammonium polyacryldimethyltauramide, sodium styrene/maleic anhydride copolymer, carbomer, and acrylates/C10-30 alkylacrylate crosspolymer.

In some embodiments, the electrical composition includes one or more viscosity increasers selected from cetyl alcohol, xanthan gum, dimethicone and dimethicone/vinyl dimethicone crosspolymer, carbomer, and acrylates/C10-30 alkylacrylate crosspolymer.

In some embodiments, the electrical composition includes one or more antioxidants selected from tocopheryl acetate, and ascorbic acid.

In some embodiments, the electrical composition includes one or more binders selected from xanthan gum.

In some embodiments, the electrical composition includes one or more skin bleaching agents selected from oxothiazolidinecarboxylic acid.

In some embodiments, the electrical composition includes one or more pH adjusters selected from triethanolamine, potassium hydroxide, and sodium hydroxide.

In some embodiments, the electrical composition includes one or more buffering agents selected from potassium hydroxide and hydroxyethylpiperazine ethane sulfonic acid, and sodium hydroxide.

In some embodiments, the electrical composition includes one or more denaturants selected from sodium hydroxide.

In some embodiments, the electrical composition includes one or more bulking agents selected from nylon-12.

In some embodiments, the electrical composition includes one or more opacifying agents selected from nylon-12.

In some embodiments, a hyaluronic acid composition herein can "comprise" the specified components, leaving open the possibility of other unspecified components.

In some embodiments, a hyaluronic acid composition herein can "consist" of the specific components, meaning the composition only includes the specified components. Stated another way, the specified components constitute 100% by weight of the hyaluronic acid composition.

### EXAMPLES

### I - Introduction

The effect of electroporation parameters on skin integrity and transport of hyaluronic acid (at 1 % by weight in water) 120 kDa (HA) labeled with fluorescein into the permeabilized skin was investigated in vitro using electroporation vs. its passive diffusion.

A voltage of 50V and 250 V was used with pulse length between 1-80 ms, pulse interval of 10-50 and number of pulses of 1-80.

This conducts to significant changes in skin impedances and thus, an increase permeability of HA 120 kDa. It has been demonstrated a significant increase in the skin deposited into the skin (x6 compared to passive diffusion).

The combination of such parameters with iontophoresis at 0.2 mA/cm² for 20 min has also been corroborated (x 12 vs. passive diffusion). Micrographs of confocal microscopy also prove such effect and the lack of significant damage to the skin.

This shows that a combined treatment of electroporation at such conditions plus iontophoresis may decrease the skin impedance and enhance the passage much importantly than electroporation alone.

### II - First experiments

Two modes have been tested:
i) transfer mode (50-99 V, longer pulse lengths and high number of pulses) and
ii) porating mode (100-250V, shorter pulse lengths and low number of pulses). Franz-diffusion cells were used to evaluate the in vitro delivery.

The experimental protocol has been as follows:
1. Skin source and treatment: Porcine ear skin was used for the in vitro studies. It is accepted as a good model for the human skin barrier. Hairs were excised from the skin surface using clippers. The skin was "sliced" so as to obtain samples with an average thickness of 1.5 mm for all samples. Skin was frozen or stored at 4°C until use.
2. Identification of optimal electroporation parameters

The following procedure was used to evaluate the effect of the electroporation parameters:
1. Leave the porcine ear skin for 1 hour at room temperature.
2. Set up the program of electroporation condition.
3. Rinse the skin well with Milli-Q water.
4. Place the skin samples in 3-compartment Franz-type vertical diffusion cells.
5. Add PBS buffer to the electrode compartments (i.e. upper compartments) to ensure good electrical contact between the electrodes and the skin.
6. Measure the skin resistance before electroporation (5 mm platinum disk electrode).
7. Apply electroporation current, as shown in the tables 1 and 2 below.
8. Measure the skin resistance after electroporation.
9. Take photos to make a visual record of skin condition.

**Transfer mode (conditions 1-9)**

| **Condition** | **Voltage (V)** | **Pulse length (ms)** | **Pulse interval (ms)** | **Pulses** |
|---|---|---|---|---|
| 1 | 99,9 | 80 | 10 | 80 |
| 2 | | 40 | 10 | 60 |
| 3 | | 20 | 10 | 40 |
| 4 | | 1 | 10 | 20 |
| 5 | 50,0 | 80 | 10 | 80 |
| 6 | | 40 | 10 | 60 |
| 7 | | 20 | 10 | 40 |
| 8 | | 1 | 10 | 20 |

**Poring mode (conditions 9-16)**

| **Condition** | **Voltage (V)** | **Pulse length (ms)** | **Pulse interval (ms)** | **Pulses** |
|---|---|---|---|---|
| 9 | 250 | 15 | 50 | 9 |
| 10 | | 10 | 50 | 6 |
| 11 | | 5 | 50 | 3 |
| 12 | | 1 | 50 | 1 |
| 13 | 150 | 15 | 50 | 9 |
| 14 | | 10 | 50 | 6 |
| 15 | | 5 | 50 | 3 |
| 16 | | 1 | 50 | 1 |

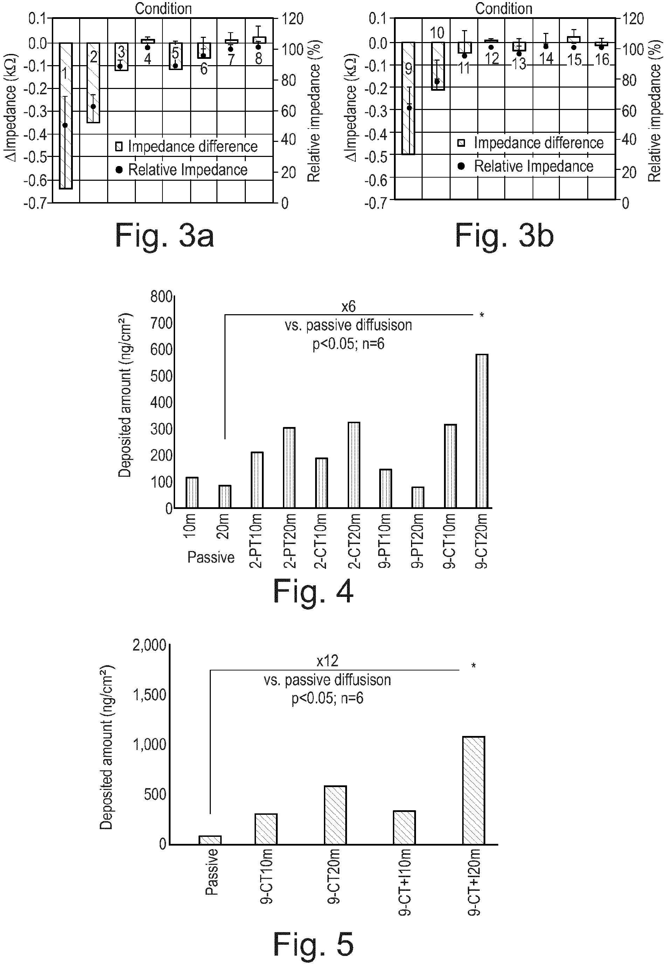

The results are shown on figures 3a and 3b.

On figure 3a is shown the impedance change (ΔImpedance = Final - Initial) and Relative Impedance (Final/Initial) in "Transfer Pulse Mode". The absolute change in impedance (ΔImpedance) is shown by the bars and the relative impedance given by the ratio of the final to the initial impedance is given by the circles. The largest decrease (or lowest relative impedance) is seen for Treatments 1 and 2 which had the most aggressive conditions (the higher voltage, the longer pulse lengths and the greater number of pulses).

On figure 3b is shown the impedance difference ((ΔImpedance = Final - Initial) and Relative Impedance (Final/Initial) for "Poring Pulse Mode". The absolute change in impedance (ΔImpedance) is shown by the bars and the relative impedance given by the ratio of the final to the initial impedance is given by the circles. As above, the largest decrease (or lowest relative impedance) is seen for the most aggressive treatments - in this case, Treatments 9 and 10.

Conditions 1, 2, 9 and 10 provide the high decrease of skin impedance.

### III - Second experiments

In order to test delivery of HA through the skin, conditions 2, 9 and 10 were selected. Application of the formulation was conducted in two different ways, previous (pre-treatment- PT) and simultaneously to the electrical treatment (co-treatment - CT), in order to obtain the optimal conditions for active delivery.

The experimental protocol has been as follows:

### 1. Skin source and treatment

Porcine ear skin was used for the in vitro studies. It is accepted as a good model for the human skin barrier. Hairs were excised from the skin surface using clippers. The skin was "sliced" so as to obtain samples with an average thickness of 1.5 mm for all samples. Skin was frozen (-20°C) or stored at 4°C until use.

### 2. Analytical method

The FL-HA was quantified using by fluorimetry. The measurements were performed using a microplate reader. The excitation and emission wavelengths were 494 and 514 nm. The gain was adjusted to 80 in order to obtain better sensitivity of the samples. All samples were put in polystyrene 96-well plates and protected from the light until analysis. Furthermore, a calibration curve was included in every plate.

### 3. Skin transport study

The aim of the experiments was to quantify penetration of FL-HA (120 kDa) into intact, electroporated porcine ear skin (with and without iontophoresis) as a function of duration using the optimal treatment conditions identified in the previous part of the study. Electroporation treatment was performed either as (i) pre-treatment, where electroporation was performed before formulation application, or as (ii) co-treatment, where the electroporation treatment was performed simultaneously in the presence of the formulation. All conditions were tested for 10 and 20 min.

The following specific conditions treatments were tested:

### TRANSFER PULSE MODE (Condition nos. from Preliminary report)

1. Condition 2 (Voltage 99.9 V, Pulse length 40 ms, Pulse interval 10 ms and 60 Pulses), pre-treatment with electroporation
2. Condition 2 (Voltage 99.9 V, Pulse length 40 ms, Pulse interval 10 ms and 60 Pulses), simultaneous treatment with electroporation and formulation

### PORING PULSE MODE

3. Condition 9 (Voltage 250 V, Pulse length 15 ms, Pulse interval 50 ms and 9 Pulses), pre-treatment with electroporation
4. Condition 9 (Voltage 250 V, Pulse length 15 ms, Pulse interval 50 ms and 9 Pulses), simultaneous treatment with electroporation and formulation
5. Condition 10 (Voltage 250 V, Pulse length 15 ms, Pulse interval 50 ms and 6 Pulses), pre-treatment with electroporation
6. Condition 10 (Voltage 250 V, Pulse length 15 ms, Pulse interval 50 ms and 6 Pulses), simultaneous treatment with electroporation and formulation
7. Control: Application of an aqueous solution of FL-HA (120 kDa) formulation (1% w/w) to "untreated" skin.

The results of the conditions 2 and 9 with passive diffusion for 10 min or 20 min (vs. single passive diffusion) are shown on figure 4. Figure 4 shows skin deposition of 120kDa FL-HA formulation following PT (Pre-Treatment; Electroporation BEFORE formulation application), CT (Co-Treatment; Electroporation AFTER formulation application).

The use of 250 V (pulse length 15; pulse interval 50 ms; 9 pulses) enhanced significantly the amount of fluorescence labeled HA 120 kDa at 1% (w/v) into the skin (x6) compared to the passive diffusion of the active. The use of higher voltages and short pulse length and low number of pulses results more efficient for skin delivery while keeping skin integrity, rather than increased number of pulses at a lower voltage.

### IV - Third experiments

The following specific conditions treatments were tested:
ELECTROPORATION + IONTOPHORESIS

Application of an aqueous solution of each FL-HA formulation (1% w/w) to "treated" skin following electroporation using previous poring pulse mode (condition 9) and iontophoresis at 0.2 mA/cm² for 10 and 20 min. Iontophoresis-combined treatment employed cathodal direct current (DC) at 0.2 mA/cm² (Ag/AgCl electrodes, DC generator).

The results are shown on figure 5. Figure 5 shows the comparison of skin deposition of 120kDa FL-HA formulation after electroporation alone (CT; Electroporation AFTER formulation application) or electroporation plus iontophoresis (**CT+I;** Electroporation AFTER formulation application followed by iontophoresis).

The comparison of the deposition when performing electroporation with and without iontophoresis showed statistical difference with the "CT+I20m" condition as compared to the rest (p<0.05; ANOVA, Student Newman Keuls test). The similar deposition obtained with 10 min electroporation, indicates that up to 10 min, iontophoresis is not increasing the deposition; nevertheless, after iontophoresis for 20 min, the increase was statistically superior.

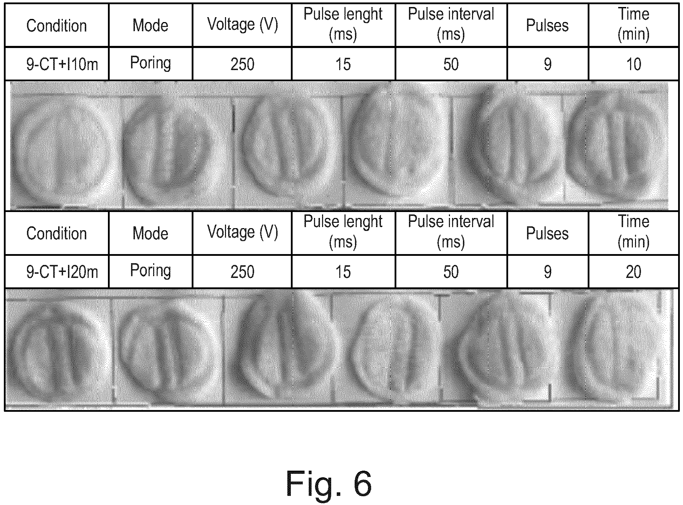

The skin images obtained provide more information about the possible skin damage produced by the electroporation treatment. In the case of condition 9 plus iontophoresis, no damage was observed, as shown on figure 6.

Co-treatment of the formulation leads to optimal conditions for electropermeabilization. When the technique is combined with iontophoresis, an important increase is observed compared to the passive diffusion of the active corroborating its efficacy on active transport of hydrophilic and charged molecules.

It has been demonstrated that electroporation of the skin by electrical shots with short pulse length of 15 ms, pulse interval of 50 ms and low number of pulses (<10) at 250 V is favorable to enhance skin deposition (x6) of HA 120 kDa compared to its passive diffusion, with no visual damage to the membrane. The effectiveness of this technique in combination with iontophoresis is also corroborated, and can be dedicated to durable treatments.

While illustrative embodiments have been illustrated and described, it will be appreciated that various changes can be made therein without departing from the spirit and scope of the claimed subject matter.

## Claims

1. An electrical method of delivering hyaluronic acid through the skin, the electrical method comprising: applying a selected electroporation pulsatile current stimulus, from any device and/or support comprising at least one electrode, the electroporation pulsatile current stimulus being of a character and for a duration sufficient to transdermally deliver hyaluronic acid, for example an aqueous composition, to a biological subject, and transporting different rates of hyaluronic acid across the skin in accordance to the selected current mode.

2. The electrical method of the preceding claim, wherein applying an electroporation pulsatile current stimulus to a biological subject includes generating an electroporation pulsatile current stimulus having a voltage below 500 V, preferably below 400 V and more preferably below 300 V.

3. The electrical method of any preceding claim, wherein applying an electroporation pulsatile current stimulus to a biological subject may include generating an electroporation pulsatile current stimulus having a voltage higher than 20 V, preferably higher than 50 V, and more preferably higher than 80 V and even higher than 100 V.

4. The electrical method of any preceding claim, wherein the electroporation pulsatile current stimulus comprises pulses having a pulse duration ranging from 3 milliseconds to 100 milliseconds, preferably from 5 milliseconds to 70 milliseconds and more preferably from 7 milliseconds to 50 milliseconds.

5. The electrical method of any preceding claim, wherein the electroporation pulsatile current stimulus comprises pulses having a pulse interval ranging from 5 milliseconds to 100 milliseconds, preferably from 5 milliseconds to 80 milliseconds and more preferably from 7 milliseconds to 60 milliseconds.

6. The electrical method of any preceding claim, wherein the electroporation pulsatile current stimulus comprises a number of pulses ranging from 1 to 200 pulses, preferably from 3 to 150 pulses, and more preferably from 5 to 100 pulses.

7. The electrical method of any preceding claim, wherein the electroporation pulsatile current stimulus includes generating a pulsed current having periodic square waveforms, rectangular waveforms, saw tooth waveforms, spiked waveforms, trapezoidal waveforms, triangle waveforms, or combinations thereof, and/or sinusoidal waveforms, non-sinusoidal waveforms, or combinations thereof.

8. The electrical method of any preceding claim, also comprising applying to a biological subject an iontophoresis direct current stimulus having an average current density ranging from 0.001 mA/cm² to 1 mA/cm², preferably from 0.01 mA/cm² to 0.5 mA/cm², and more preferably from 0.05 mA/cm² to 0.3 mA/cm², and in particular up to or below 0.2 mA/cm².

9. The electrical method of the preceding claim, wherein the iontophoresis direct current stimulus is applied for a duration ranging from 1 minute to 60 minutes, preferably from 5 minutes to 50 minutes.

10. The electrical method of any preceding claim, further comprising: transdermally delivering a composition including one or more of hyaluronic acid, hyaluronic acid derivatives, ions of hyaluronic acid, and ions of hyaluronic acid derivatives present in amounts ranging from 0.01 % to 100 % by weight, preferably from 0.5 % to 60 % by weight.

11. The electrical method of the preceding claim, wherein the hyaluronic acid has a molecular weight in a range from 1 Da to 4000 kDa, preferably from 1 kDa to 2000 kDa and more preferably from 5 kDa to 200 kDa.

12. The electrical method according to any preceding claims, comprising the step of measuring at least one of the temperature of the skin, the impedance of the skin, and a pH of the composition, and wherein the application of current stimulus may be reduced to a safety level when a measured value measured by one of the sensors exceeds a safety range or a safety value.

13. An electrical composition, comprising:
one or more of hyaluronic acid, hyaluronic acid derivatives, ions of hyaluronic acid, and ions of hyaluronic acid derivatives present in amounts ranging from 0.01 % to 30 % by weight, preferably in an amounts ranging from 0.1 % to 20 % by weight, 0.1% to 5 % by weight, and
water present in an amount of at least 0 % by weight, preferably in an amount of at least 10 % by weight, more preferably in an amount of at least 60 % by weight, more preferably in an amount of at least 90 % by weight.

14. An electrical kit comprising:
- An electrical composition including one or more of hyaluronic acid, hyaluronic acid derivatives, ions of hyaluronic acid, and ions of hyaluronic acid derivatives, and
- An electrical device for carrying the electrical method of anyone of claims 1 to 11.

15. The electrical kit of the preceding claim, wherein the composition is an aqueous composition.

16. The electrical kit of any one of the two preceding claims, the kit being configured such that hyaluronic acid and water are already mixed in the composition when the composition is applied to the skin.

17. The electrical kit of any one of claims 14 to 16, the device comprising at least one of a temperature sensor, an impedance sensor, and a pH sensor, and wherein the device may be configured such that the application of current stimulus is reduced to a safety level when a measured value measured by one of the sensors exceeds a safety range or a safety value.
